# EUROPÄISCHE PATENTSCHRIFT

(11) **EP 1 713 762 B1**
(45) Veröffentlichungstag und Bekanntmachung des Hinweises auf die Patenterteilung: **13.04.2011**
(21) Anmeldenummer: 05707028.6
(22) Anmeldetag: 27.01.2005
(51) Int. Cl.: C07C 253/10, C07C 255/07

(54) **VERFAHREN ZUR HYDROCYANIERUNG VON BUTADIEN**
METHOD FOR THE HYDROCYANATION OF BUTADIENE
PROCEDE D'HYDROCYANATION DE BUTADIENE

(30) Priorität: 29.01.2004 DE 102004004673
(43) Veröffentlichungstag der Anmeldung: 25.10.2006
(73) Patentinhaber: BASF SE, 67056 Ludwigshafen (DE)
(72) Erfinder: JUNGKAMP, Tim, B-2950 Kapellen (BE); BAUMANN, Robert, 68159 Mannheim (DE); BARTSCH, Michael, 67433 Neustadt (DE); HADERLEIN, Gerd, 67269 Grünstadt (DE); LUYKEN, Hermann, 67069 Ludwigshafen (DE); SCHEIDEL, Jens, 69493 Hirschberg (DE)
(86) Internationale Anmeldenummer: PCT/EP2005/000780
(87) Internationale Veröffentlichungsnummer: WO 2005/073173

(56) Entgegenhaltungen:
- WO-A-99/07671
- US-A- 3 850 973
- WALTER BRÖTZ; AXEL SCHÖNBUCHER: "Technische Chemie I (Grundverfahren)" 1982, VERLAG CHEMIE , WEINHEIM; DEEFIELD BEACH, FLORIDA; BASEL , XP002328711 Seiten 157, 158, 246, 247

## Beschreibung

Die vorliegende Erfindung betrifft ein Verfahren zur Herstellung von 3-Pentennitril durch Hydrocyanierung von 1,3-Butadien in Gegenwart mindestens eines Katalysators.

Adipodinitril ist ein wichtiges Ausgangsprodukt in der Nylonherstellung, das durch zweifache Hydrocyanierung von 1,3-Butadien erhalten wird, wie z.B. in WO 99/07671 beschrieben. Dabei wird in einer ersten Hydrocyanierung 1,3-Butadien zu 3-Pentennitril hydrocyaniert. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Phosphor-Komplexe katalysiert.

Wenn die Konzentration an Cyanwasserstoff während der Hydrocyanierung von 1,3-Butadien zu groß ist, kommt es zu einer Bildung von Nickel(II)-cyaniden. Die Bildung dieser Nickel(II)-salze ist auf eine Oxidation des Nickel(0)-katalysators mit Cyanwasserstoff zurückzuführen.

Casalnuovo beschreibt in J. Am. Chem. Soc. 116, Seite 9.869 (1994) einen Reaktionsmechanismus für die Hydrocyanierung von Olefinen unter Bildung von Nickel(II)-cyanid durch Oxidation des Nickel(0)-katalysators mit Cyanwasserstoff in einer Parallelreaktion.

Aus den bekannten Verfahren zur Herstellung von 3-Pentennitril ist nicht bekannt, wie während der Hydrocyanierung von 1,3-Butadien die Konzentration an Cyanwasserstoff so niedrig gehalten werden kann, dass einerseits eine ausreichende Reaktionsgeschwindigkeit der Hydrocyanierung sichergestellt wird und andererseits die Bildung von Nickel(II)-cyaniden vermieden werden kann.

Die Aufgabe der vorliegenden Erfindung ist somit, ein Verfahren zur Herstellung von 3-Pentennitril durch Hydrocyanierung von 1,3-Butadien in Gegenwart mindestens eines Katalysators bereitzustellen, bei dem eine ausreichende Hydrocyanierungsgeschwindigkeit erreicht wird und gleichzeitig die Bildung von Nickel(II)-cyaniden vermieden wird.

Die Lösung dieser Aufgabe geht aus von einem Verfahren zur Herstellung von 3-Pentennitril durch Hydrocyanierung von 1,3-Butadien in Gegenwart mindestens eines Katalysators. Das erfindungsgemäße Verfahren ist dann dadurch gekennzeichnet, dass die Hydrocyanierung in einem Schlaufenreaktor mit mindestens einer Zuleitung und mindestens einer Ableitung, einem externem Umpumpkreislauf, einem Einleitrohr und mindestens einer Strahldüse zum Antrieb der inneren Zirkulation durchgeführt wird. Durch das Umpumpen wird der nötige Vordruck für die Verdüsung über die Strahldüse erzeugt. In einer bevorzugten Ausführungsform ist der Umpumpkreis so gestaltet, dass das Volumen der Rohrleitungen und Apparate im Umpumpkreislauf als Nachreaktionszone genutzt werden kann.

Die Verwendung eines Schlaufenreaktors in einer Hydrocyanierung von 1,3-Butadien ist wegen der kurzen Einmischzeiten und dem damit verbundenen geringen Umfang an lokalen Überschusskonzentrationen von Cyanwasserstoff vorteilhaft, weil die Nickelcyanidbildungsrate gegenüber der Produktbildungsrate abnimmt.

In einer Ausführungsform der vorliegenden Erfindung wird das erfindungsgemäße Verfahren kontinuierlich durchgeführt.

In einer weiteren Ausführungsform der vorliegenden Erfindung wird das erfindungsgemäße Verfahren in flüssiger Phase durchgeführt. Daher wird der Druck im Reaktor vorzugsweise so eingestellt, dass unter der verwendeten Reaktionstemperatur die Reaktanden in flüssiger Form vorliegen. Ferner ist es bevorzugt, dass das 1,3-Butadien und der Cyanwasserstoff in flüssiger Form in das erfindungsgemäße Verfahren eingesetzt werden.

In einer weiteren Ausführungsform des erfindungsgemäßen Verfahrens wird der Schlaufenreaktor geflutet gefahren. Unter einer gefluteten Fahrweise wird verstanden, dass die Eindüsung in die Flüssigphase erfolgt, und eine Gasphase in den Reaktor nicht eingemischt wird, so dass im Reaktionsvolumen kein wesentlicher Anteil an Gasphase, vorzugsweise keine Gasphase vorhanden ist, und vorzugsweise im Inneren des Schlaufenreaktors keine Gasphase vorhanden ist. Eine geflutete Fahrweise in dem erfindungsgemäßen Verfahren ist bevorzugt, da bei einer zweiphasigen Fahrweise 1,3-Butadien in der Flüssigphase abgereichert wird. Damit wird Cyanwasserstoff angereichert und es bildet sich vermehrt Nickel(II)-cyanid.

Für Hydrocyanierungen können ein oder mehrere Reaktoren verwendet werden, die bei Verwendung von mehreren Reaktoren vorzugsweise in Reihe geschaltet werden.

In einer Ausführungsform des erfindungsgemäßen Verfahrens wird neben dem Schlaufenreaktor ein oder mehrere weitere Reaktoren verwendet, wobei mindestens zwei Reaktoren in Reihe geschaltet sind, Cyanwasserstoff in mehr als einen Reaktor und 1,3-Butadien und der mindestens eine Katalysator in den ersten der in Reihe geschalteten Reaktoren eingeführt werden.

In einer Ausführungsform des erfindungsgemäßen Verfahrens wird nur ein Reaktor verwendet, in den Cyanwasserstoff, 1,3-Butadien und der mindestens eine Katalysator dosiert werden.

In dem erfindungsgemäßen Verfahren können einer oder mehrere dieser Reaktoren Schlaufenreaktoren sein, besonders bevorzugt Reaktoren in die Cyanwasserstoff eingeleitet wird.

1,3-Butadien, der mindestens eine Katalysator und Cyanwasserstoff können über getrennte Zuläufe in die innere Schlaufe des erfindungsgemäß verwendeten Schlaufenreaktors dosiert werden. Dabei wird Cyanwasserstoff bevorzugt über die Strahldüse dosiert. Der in dem erfindungsgemäßen Verfahren verwendete Schlaufenreaktor ist besonders bevorzugt mit einer Düse ausgestattet, die einen Zulauf für Cyanwasserstoff und einen Zulauf für den externen Kreislaufstrom aufweist. Dadurch ergibt sich die Möglichkeit, dass 3-Pentennitril als Umsetzungsprodukt des Reaktors an der Stelle entnommen werden kann, wo der interne Zirkulationsstrom die längste Umlaufzeit vor dem Vermischen mit dem Treibstrahl hat.

Darüber hinaus ist es bevorzugt, dass der Cyanwasserstoff in einem inneren Einleitrohr geführt wird und der Umpumpstrom koaxial um dieses Einleitrohr herumgeführt wird, da dies eine sehr gute Vermischung der Reaktanden ohne lokale Überkonzentrationen an Cyanwasserstoff gegenüber dem im Umpumpstrom enthaltenen mindestens einen Katalysator ergibt. Diese Überkonzentrationen würden wiederum zu einer erhöhten Bildungsrate an Nickel(II)cyanid-Verbindungen führen.

In einer weiteren Ausführungsform des erfindungsgemäßen Verfahrens wird das 1,3-Butadien und/oder der mindestens eine Katalysator in den externen Umpumpkreis des Schlaufenreaktors eindosiert.

In einer weiteren Ausführungsform des erfindungsgemäßen Verfahrens ist der in dem erfindungsgemäßen Verfahren verwendete Schlaufenreaktor vertikal gebaut und die Düse oben eingebaut.

Die Einleitstelle zum Eindosieren von Cyanwasserstoff in den Schlaufenreaktor ist vorzugsweise gekühlt.

Um Fouling in nachgeschalteten Verfahrensstufen zu vermeiden, ist es bevorzugt, dass dem Schlaufenreaktor ein Nachreaktor nachgeschaltet ist, der vorzugsweise eine Rohrcharakteristik aufweist.

Das erfindungsgemäße Verfahren umfasst die Umsetzung von 1,3-Butadien und Cyanwasserstoff an mindestens einem Katalysator. Als Katalysatoren werden Nickel(0)-Katalysator-Komplexe verwendet.

Bei den Ni(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden sind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel Ia

(o-Tolyl-O-)_{w} (m-Tolyl-O-)ₓ (p-Tolyl-O-)_{y} (Phenyl-O-)_{z} P (I a)

eingesetzt werden, wobei w, x, y und z eine natürliche Zahl bedeuten, und folgende Bedingungen gelten: w + x + y + z = 3 und w, z ≤ 2.

Solche Verbindungen Ia sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P , (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Totyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z} (O-R⁴)ₚ (I b)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der a- romatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x:: 1 oder 2,
- y, z, p:: unabhängig voneinander 0, 1 oder 2 mit der Maßgabe, dass x+y+z+p = 3.

Bevorzugte Phosphite der Formel Ib sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung Ib ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel Ib sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel Ib sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel Ib können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel Ib.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite Ib und zur Aufarbeitung sind der DE-A 199 53 058 zu entnehmen.

Die Phosphite Ib können auch in Form eines Gemisches verschiedener Phosphite Ib als Ligand verwendet werden. Ein solches Gemisch kann beispielsweise bei der Herstellung der Phosphite Ib anfallen.

Es ist allerdings bevorzugt, dass der phosphorhaltige Ligand mehrzähnig, insbesondere zweizähnig ist. Daher weist der verwendete Ligand vorzugsweise die Formel II 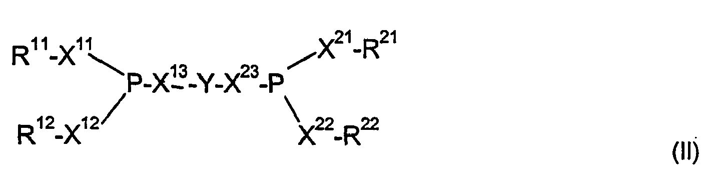 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, Ia, Ib und II sowie deren Herstellung sind an sich bekannt. Als phosphorhaltiger Ligand können auch Mischungen, enthaltend mindestens zwei der Verbindungen I, Ia, Ib und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel Ib

P (O-R¹)ₓ (O-R²)_{y} (O-R³)_{z} (O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen. Die Hydrocyanierung wird vorzugsweise bei Drücken von 0,1 bis 500 MPa, besonders bevorzugt 0,5 bis 50 MPa, insbesondere 1 bis 5 MPa durchgeführt. Die Reaktion wird vorzugsweise bei Temperaturen von 273 bis 473 K, besonders bevorzugt 313 bis 423 K, insbesondere bei 333 bis 393 K durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, pro Reaktor als vorteilhaft erwiesen.

1,3-Butadien kann molar im Verhältnis 1:1 im Vergleich zu Cyanwasserstoff bezogen auf die Summe aller Zuläufe der Reaktorkaskade, dosiert werden. Es ist allerdings bevorzugt, bei der Reaktionsführung 1,3-Butadien so zu dosieren, dass die molaren Mengenverhältnisse zwischen 1,3-Butadien zu Cyanwasserstoff im Bereich von 1,6 : 1 bis 1,1 : 1, besonders bevorzugt 1,6 : 1 bis 1,3 : 1 liegen, wie in der DE-A-102 004 004 696 beschrieben. Bei Aufteilung des Cyanwasserstoffs in gleichen Teilen auf mehrere Reaktoren ergeben sich daraus für den einzelnen Reaktor betrachtet höhere Überschüsse an 1,3-Butadien, die durch die oben stehenden Angaben nicht ausgenommen sind.

Die Auslegung des Schlaufenreaktors in seiner Größe wird vorzugsweise so vorgenommen, dass der Restgehalt an Cyanwasserstoff im Austragsstrom aus dem Schlaufenreaktor kleiner als 10 Gew.-%, besonders bevorzugt kleiner als 5 Gew.-%, insbesondere kleiner als 1 Gew.-%, bezogen auf die Masse des Austragsstromes, beträgt. Diese Angaben gelten bei den Zielumsätzen bzgl. 1,3-Butadien, bevorzugt auch bei niedrigeren Umsätzen als dem Zielumsatz, wenn der Katalysator zum Teil desaktiviert ist.

Die Verwendung eines Schlaufenreaktors in einem Verfahren zur Herstellung von 3-Pentennitril ist wegen der kurzen Einmischzeiten und dem damit verbundenen geringen Umfang an lokalen Überschusskonzentrationen von Cyanwasserstoff bevorzugt. Hierdurch wird die Nickelcyanidbildungsrate gegenüber der Produktbildungsrate herabgesetzt. Zudem kann die Reaktionsenergie aus dem vorliegenden System leicht abgeführt werden, da der Schlaufenreaktor erfindungsgemäß mit mindestens einen Wärmeübertrager im externen Umlauf ausgestattet ist.

### Ausführungsbeispiele:

Im Folgenden wird in Ausführungsbeispielen der Vorteil von Strahlschlaufenreaktoren hinsichtlich der geringen Austrittskonzentration an Cyanwasserstoff exemplarisch aufgezeigt.

In allen Beispielen wird ein Strahlschlaufenreaktor mit einem Volumen von 12 I verwendet. Das innere Rohr hat einen Durchmesser von 50 mm und eine Länge von 1,4 m. Der Umpumpkreislauf besteht aus einer Pumpe und einem Wärmeübertrager, der als Kühler zur Abfuhr der Reaktionswärme verwendet wird. Die Apparate und die Leitung des Umpumpkreises haben ein Volumen von zusammen 20 l.

Cyanwasserstoff wird über eine auf 0°C gekühlte Treibstrahldüse am Kopf des vertikal gebauten Schlaufenreaktors eingedüst, 1,3-Butadien und der Katalysatorstrom werden in den Umpumpkreis mit einer Temperatur von 100°C eingeleitet. Die Entnahme des Produktstromes erfolgt aus dem äußeren Schenkel des internen Umlaufs bei ca. 75% der Höhe des Reaktors. Der Umpumpkreislaufstrom wird am unteren Ende des Reaktors abgezogen und über die Düse wieder eingespeist. Zwischen diesen beiden Punkten wird eine Temperaturdifferenz gemessen, die ein Maß für die Reaktionsrate im Inneren des Schlaufenreaktors ist. Die Temperatur am unteren Ausgang des Reaktors wird durch den Wärmeabzug im externen Kühler geregelt.

Als stabilisierende Liganden des Nickel(0)-Komplexes wird ein Gemisch aus dem Chelatliganden 1 und Tri-m/p-tolylphosphit verwendet. Die Katalysatorlösung wird erzeugt, indem man 40 kg einer Mischung mit 2,3 Gew.-% Nickel(0), 15 Gew.-% 3-Pentennitril und dem Rest Tri-m/p-tolylphosphit mit 104 kg einer anderen Mischung mit 23,1 % des Liganden 1 und dem Rest 3-Pentennitril abmischt unter Erhalt einer Lösung, die im folgenden mit Katalysatorlösung bezeichnet wird.

Aus dem Austragsstrom aus dem Schlaufenreaktor werden Proben gezogen. Durch Absorption des Cyanwasserstoffs in auf 0 °C gekühlter wässriger Natronlauge wird der Gehalt des Stromes an Cyanwasserstoff quantitativ entfernt und durch unmittelbar anschließende Titration der Natronlauge bestimmt.

### Abkürzungen:

HCN = Cyanwasserstoff
BD = 1,3-Butadien
KAT = Katalysatorlösung
ΔT = Temperaturdifferenz zwischen Entnahmestelle Umpumpkreislaufstrom und Zulaufstelle Umpumpkreislaufstrom

### Beispiel 1:

Dem Reaktor werden 7,5 kg/h HCN zugeführt, in den Umlaufkreis werden 18,0 kg/h BD und 11,7 kg/h KAT dosiert. Es fällt ein Produktstrom von 37,2 kg/h an. ΔT beträgt nach 48 h Laufzeit 4 K. Die Analyse zu diesem Zeitpunkt ergibt einen Gehalt von HCN im Austragsstrom von 90 Gew.-ppm.

Dieses Beispiel illustriert die geringe HCN-Konzentration, die sich im aufzuarbeitenden Produktstrom ergibt. Der Umsatz des Butadiens ist dabei hoch im Vergleich zu Beispiel 2.

### Beispiel 2:

Dem Reaktor werden 7,5 kg/h HCN zugeführt, in den Umlaufkreis werden 18,0 kg/h BD und 2,6 kg/h KAT dosiert. Es fällt ein Produktstrom von 28,1 kg/h an. ΔT beträgt nach 46 h Laufzeit 2 K. Die Analyse zu diesem Zeitpunkt ergibt einen Gehalt von HCN im Austragsstrom von 1470 Gew.-ppm.

Dieses Beispiel illustriert, dass auch bei niedrigerem Umsatz, der sich durch die höhere Katalysatorbelastung als in Beispiel 1 ergibt, der aufzuarbeitende Produktstrom nur eine geringe Menge HCN enthält.

### Beispiel 3:

Dem Reaktor werden 7,5 kg/h HCN zugeführt, in den Umlaufkreis werden 21,0 kg/h BD und 31,7 kg/h KAT dosiert. Es fällt ein Produktstrom von 60,2 kg/h an. Der Austragsstrom erfuhr nach dem Austritt aus dem Reaktor eine Aufarbeitung, wie sie in Beispiel 1 der DE-A-102 004 004 724 beschrieben ist. Der Dabei anfallende Katalysator-Rückführstrom wurde ohne Ausschleusung und Frischkatalysatorergänzung in der oben beschriebenen Menge zurückgefahren. Die Menge an Butadien enthält ebenso das auf diese Weise zurückgeführte Butadien, das um die entsprechend nötige Menge Frischbutadien ergänzt wurde.

Nach 2 h Laufzeit beträgt ΔT 5,5 K. Die Analyse zu diesem Zeitpunkt ergibt einen Gehalt von HCN im Austragsstrom von kleiner 10 Gew.-ppm. Nach 126 h Laufzeit beträgt ΔT 2,5 K. Die Analyse zu diesem Zeitpunkt ergibt einen Gehalt von HCN im Austragsstrom von 2260 Gew.-ppm.

Das Beispiel zeigt, dass auch bei einbrechendem Umsatz infolge von Katalysatordesaktivierung in einem geschlossenen Katalysatorkreislauf der HCN-Gehalt im auszuarbeitenden Produktstrom niedrig bleibt.

## Patentansprüche

1. Verfahren zur Herstellung von 3-Pentennitril durch Hydrocyanierung von 1,3-Butadien in Gegenwart mindestens eines Ni(O)-Komplexes als Katalysator, der phosphorhaltige Liganden, ausgewählt aus mono- und bidentaten Phosphinen, Phosphiten, Phosphiniten und Phosphoniten enthält, **dadurch gekennzeichnet, dass** die Hydrocyanierung in einem Schlaufenreaktor mit mindestens einer Zuleitung und mindestens einer Ableitung, einem externen Umpumpkreislauf, einem Einleitrohr und mindestens einer Strahldüse zum Antrieb der inneren Zirkulation durchgeführt wird.

2. Verfahren nach Anspruch 1, **dadurch gekennzeichnet, dass** das Verfahren kontinuierlich durchgeführt wird.

3. Verfahren nach Anspruch 1 oder 2, **dadurch gekennzeichnet, dass** das Verfahren in flüssiger Phase durchgeführt wird.

4. Verfahren nach einem der Ansprüche 1 bis 3, **dadurch gekennzeichnet, dass** der Schlaufenreaktor geflutet gefahren wird.

5. Verfahren nach einem der Ansprüche 1 bis 4, **dadurch gekennzeichnet, dass** für die Hydrocyanierung neben dem Schlaufenreaktor ein oder mehrere weitere Reaktoren verwendet werden, wobei mindestens zwei Reaktoren in Reihe geschaltet sind, Cyanwasserstoff in mehr als einen Reaktor und 1,3-Butadien und der mindestens eine Katalysator in den ersten der in Reihe geschalteten Reaktoren eingeführt werden.

6. Verfahren nach einem der Ansprüche 1 bis 5, **dadurch gekennzeichnet, dass** der Cyanwasserstoff in dem Schlaufenreaktor in einem inneren Einleitrohr geführt wird und der Umpumpstrom koaxial um dieses Einleitrohr herumgeführt wird.

7. Verfahren nach einem der Ansprüche 1 bis 6, **dadurch gekennzeichnet, dass** 3-Pentennitril an der Stelle des Schlaufenreaktors entnommen wird, wo der interne Zirkulationsstrom die längste Umlaufzeit vor dem Vermischen mit dem Treibstrahl hat.

8. Verfahren nach einem der Ansprüche 1 bis 7, wobei 1,3-Butadien und/oder der mindestens eine Katalysator in den externen Umpumpkreis eindosiert werden.

9. Verfahren nach einem der Ansprüche 1 bis 8, **dadurch gekennzeichnet, dass** die Einleitstelle zur Dosierung des Cyanwasserstoffs gekühlt ist.

10. Verfahren nach einem der Ansprüche 1 bis 9, **dadurch gekennzeichnet, dass** dem Schlaufenreaktor ein Nachreaktor mit Rohrcharakteristik nachgeschaltet ist.

## Claims

1. A process for preparing 3-pentenenitrile by hydrocyanating 1,3-butadiene in the presence of at least one Ni(0) complex as a catalyst comprising phosphorus ligands selected from mono- and bidentate phosphines, phosphites, phosphinites and phosphonites, which comprises carrying out the hydrocyanation in a loop reactor having at least one feed line and at least one discharge line, an external pumped circulation system, an inlet tube and at least one jet nozzle for driving the internal circulation.

2. The process according to claim 1, which is carried out continuously.

3. The process according to claim 1 or 2, which is carried out in the liquid phase.

4. The process according to any of claims 1 to 3, wherein the loop reactor is in flooded operation.

5. The process according to any of claims 1 to 4, wherein one or more additional reactors are used for the hydrocyanation in addition to the loop reactor, in which case at least two reactors are connected in series, hydrogen cyanide is introduced into more than one reactor, and 1,3-butadiene and the at least one catalyst are introduced into the first of the reactors connected in series.

6. The process according to any of claims 1 to 5, wherein the hydrogen cyanide is conducted within the loop reactor in an internal inlet tube and the pumped circulation stream is conducted coaxially aground this inlet tube.

7. The process according to any of claims 1 to 6, wherein 3-pentenenitrile is withdrawn at the point in the loop reactor where the internal circulation stream has the longest circulation time before the mixing with the driving jet.

8. The process according to any of claims 1 to 7, in which 1,3-butadiene and/or the at least one catalyst are metered into the external pumped circuit.

9. The process according to any of claims 1 to 8, wherein the inlet point for metering the hydrogen cyanide is cooled.

10. The process according to any of claims 1 to 9, wherein a postreactor having tubular characteristics is connected downstream of the loop reactor.

## Revendications

1. Procédé de fabrication de 3-pentène-nitrile par hydrocyanation de 1,3-butadiène en présence d'au moins un complexe de Ni(O) en tant que catalyseur, qui contient des ligands contenant du phosphore choisis parmi les phosphines, phosphites, phosphinites et phosphonites mono- et bidentates, **caractérisé en ce que** l'hydrocyanation est réalisée dans un réacteur à boucle comprenant au moins une conduite d'entrée et au moins une conduite de sortie, un circuit de pompage externe, un tube d'alimentation et au moins une buse à jet pour l'actionnement de la circulation intérieure.

2. Procédé selon la revendication 1, **caractérisé en ce que** le procédé est réalisé en continu.

3. Procédé selon la revendication 1 ou 2, **caractérisé en ce que** le procédé est réalisé en phase liquide.

4. Procédé selon l'une quelconque des revendications 1 à 3, **caractérisé en ce que** le réacteur à boucle est exploité à l'état engorgé.

5. Procédé selon l'une quelconque des revendications 1 à 4, **caractérisé en ce qu'**un ou plusieurs réacteurs supplémentaires sont utilisés en plus du réacteur à boucle pour l'hydrocyanation, au moins deux réacteurs étant connectés en série, du cyanure d'hydrogène étant introduit dans plus d'un réacteur et du 1,3-butadiène et le ou les catalyseurs dans le premier des réacteurs connectés en série.

6. Procédé selon l'une quelconque des revendications 1 à 5, **caractérisé en ce que** le cyanure d'hydrogène est introduit dans un tube d'alimentation intérieur dans le réacteur à boucle et le courant pompé est mis en circulation co-axialement autour de ce tube d'alimentation.

7. Procédé selon l'une quelconque des revendications 1 à 6, **caractérisé en ce que** du 3-pentène-nitrile est soutiré à l'emplacement du réacteur à boucle où le courant de circulation intérieur a le temps de circulation le plus long avant le mélange avec le jet d'entraînement.

8. Procédé selon l'une quelconque des revendications 1 à 7, dans lequel du 1,3-butadiène et/ou le ou les catalyseurs sont introduits dans le circuit de pompage externe.

9. Procédé selon l'une quelconque des revendications 1 à 8, **caractérisé en ce que** l'emplacement d'alimentation pour l'introduction du cyanure d'hydrogène est refroidi.

10. Procédé selon l'une quelconque des revendications 1 à 9, **caractérisé en ce qu'**un réacteur secondaire à caractéristique de tube est connecté en aval du réacteur à boucle.
